# EUROPEAN PATENT SPECIFICATION

(11) **EP 4 382 024 B1**
(45) Date of publication and mention of the grant of the patent: **30.09.2026**
(21) Application number: 22903356.8
(22) Date of filing: 05.12.2022
(51) Int. Cl.: A61B 1/005, A61B 1/00

(54) **BENDING SECTION AND INSERTION TUBE CONNECTING STRUCTURE, AND ENDOSCOPE**
BIEGEABSCHNITT UND EINFÜHRROHRVERBINDUNGSSTRUKTUR SOWIE ENDOSKOP
SECTION DE COURBURE ET STRUCTURE DE RACCORDEMENT DE TUBE D'INSERTION, ET ENDOSCOPE

(30) Priority: 07.12.2021 CN 202123058884 U
(43) Date of publication of application: 12.06.2024
(73) Proprietor: Guangzhou Red Pine Medical Instrument Co., Ltd., Guangzhou, Guangdong 510000 (CN)
(72) Inventor: YI, Feng, Guangzhou, Guangdong 510000 (CN); LI, Jing, Guangzhou, Guangdong 510000 (CN); TAN, Xiaofeng, Guangzhou, Guangdong 510000 (CN)
(74) Representative: Murgitroyd & Company
(86) International application number: PCT/CN2022/136470
(87) International publication number: WO 2023/103934

(56) References cited:
- CN-B- 102 835 944
- CN-U- 208 582 381
- CN-U- 211 093 923
- CN-U- 212 521 712
- CN-U- 217 040 070
- CN-U- 217 040 075
- JP-A- 2009 011 442
- JP-U- H0 613 801
- US-A1- 2009 093 679
- US-A1- 2020 154 981
- US-A1- 2020 196 835

## Description

### TECHNICAL FIELD

The present utility model relates to the technical field of medical devices, and in particular to a connecting structure for a bending section and an insertion tube, and an endoscope.

### BACKGROUND

With the development of medical technology, endoscope technology has emerged. Endoscopes are configured to visually inspect hard-to-reach locations such as organs in the human body to observe pathological changes. Typically, the endoscope includes a long insertion tube having a handle at an end of the insertion tube adjacent to an operator, and a visual inspection device such as a built-in camera at a distal end of the long insertion tube. In order to be able to control the endoscope inside the human body, the endoscope includes a bendable bending section, and an orientation of a tip of an end of the bending section is adjusted through the bending of the bending section, thereby changing an observation angle.

In conventional technology, the insertion tube is a braided tube, and the insertion tube is connected to the plastic bending section by adhesion. However, due to the extremely poor adhesive property of the material used for the bending section, the adhesive method is likely to cause the bending section to fall off from the insertion tube, and the adhesion effect is poor, which is easy to cause the bending section to fall off from the insertion tube and remain in the human organ, resulting in medical accidents.

A coupling structure for an insertion tube and a bending tube of an endoscope is disclosed in prior art document US2009093679 A1.

Accordingly, it is necessary to provide a connecting structure for a bending section and an insertion tube, and an endoscope, which can effectively improve the connection stability of the bending section and the insertion tube, prevent the bending section from falling off, and reduce the probability of medical accidents.

A connecting structure for a bending section and an insertion tube includes the bending section provided with a first engaging portion, and the first engaging portion being located in a circumferential direction of the bending section; a connecting tube sleeved with the bending section and provided with a second engaging portion, the second engaging portion being located in a circumferential direction of the connecting tube, the first engaging portion being engaged with the second engaging portion to enable the bending section to be connected to the connecting tube; the insertion tube adhered to the connecting tube.

According to the aforementioned connecting structure for the bending section and the insertion tube, during the mounting process, the connecting tube is sleeved on the bending section, the first engaging portion is engaged with the second engaging portion, and the insertion tube is adhered to the connecting tube. Since the first engaging portion is provided in the circumferential direction of the bending section and the second engaging portion is provided in the circumferential direction of the connecting tube, the connecting tube is sleeved on the bending section while being connected to the bending section through engagement, which is beneficial to reduce the chances of the first engaging portion and the second engaging portion being broken by external force, and ensure the overall structural stability of the connecting structure for the bending section and the insertion tube. At the same time, by adhering the connecting tube to the insertion tube, the bending section is connected to the insertion tube in an indirect manner, which is conducive to avoiding the risk of the bending section falling off due to poor direct bonding stability, and further conducive to ensuring the connection stability between the bending section and the insertion tube, which reduces the probability of medical accidents caused by the bending section falling off from the insertion tube when an endoscope is in use, thereby improving the overall use quality of the endoscope.

In one of the embodiments, at least two first engaging portions and at least two second engaging portions are provided, the at least two first engaging portions are spaced apart along the circumferential direction of the bending section, the at least two second engaging portions are spaced apart along a circumferential direction of the connecting tube, and the at least two first engaging portions are in one-to-one correspondence with the at least two second engaging portions.

In one of the embodiments, the first engaging portion is an engaging post, the second engaging portion is an engaging hole, and a cross-sectional profile of the engaging post is matched with a shape of an opening of the engaging hole.

In one of the embodiments, the first engaging portion is an engaging hole, the second engaging portion is an engaging post, and a shape of an opening of the engaging hole is matched with a cross-sectional profile of the engaging post.

In one of the embodiments, the engaging post is provided with a guiding inclined surface configured to guide the engaging post to enter the engaging hole.

In one of the embodiments, the at least two of the engaging posts at least include a first engaging post and a second engaging post, the at least two engaging holes at least include a first engaging hole and a second engaging hole, the first engaging post is engaged with the first engaging hole, the second engaging post is engaged with the second engaging hole, a cross-sectional profile of the first engaging post is different from a cross-sectional profile of the second engaging post, and a shape of an opening of the first engaging hole is different from a shape of an opening of the second engaging hole.

In one of the embodiments, the cross-sectional profile of the first engaging post is oblong-shaped extending along an axial direction of the bending section, and the opening of the first engaging hole is oblong-shaped extending along an axial direction of the connecting tube, the cross-sectional profile of the second engaging post is circular, and the opening of the second engaging hole is circular.

According to the invention, the bending section is provided with a first annular connecting portion connected to the bending section and extending along an axial direction of the bending section, the first engaging portion is located in a circumferential direction of the first annular connecting portion, the connecting tube is provided with a second annular connecting portion connected to the connecting tube and extending along an axial direction of the connecting tube, the second engaging portion is located in a circumferential direction of the second annular connecting portion, an outer diameter of the second annular connecting portion is less than an inner diameter of the first annular connecting portion, the first annular connecting portion is sleeved on the second annular connecting portion, the connecting tube is engaged with the first annular connecting portion, and an outer wall of the connecting tube is in smooth transition with an outer wall of the first annular connecting portion.

According to the invention, a sealant is coated between the outer wall of the second annular connecting portion and the inner wall of the first annular connecting portion.

According to the invention, the bending section is provided with a first annular connecting portion connected to the bending section and extending along an axial direction of the bending section, the first engaging portion is located in a circumferential direction of the first annular connecting portion, the connecting tube is provided with a second annular connecting portion connected to the connecting tube and extending along an axial direction of the connecting tube, the second engaging portion is located in a circumferential direction of the second annular connecting portion, an outer diameter of the first annular connecting portion is less than an inner diameter of the second annular connecting portion, the second annular connecting portion is sleeved on the first annular connecting portion, the bending section is engaged with the second annular connecting portion, and an outer wall of the bending section is in smooth transition with an outer wall of the second annular connecting portion.

According to the invention, a sealant is coated between the outer wall of the first annular connecting portion and the inner wall of the second annular connecting portion.

In one of the embodiments, the connecting tube is further provided with a third annular connecting portion connected to the connecting tube and extending along the axial direction of the connecting tube, the second annular connecting portion and the third annular connecting portion are located at opposite ends of the connecting tube, respectively, the insertion tube is provided with a fourth annular connecting portion connected to the insertion tube and extending along the axial direction of the insertion tube, an outer diameter of the fourth annular connecting portion is less than an inner diameter of the third annular connecting portion, the third annular connecting portion is sleeved on the fourth annular connecting portion, the insertion tube is engaged with the third annular connecting portion, the outer wall of the insertion tube is in smooth transition with an outer wall of the third annular connecting portion, and a structural adhesive is coated between the outer wall of the fourth annular connecting portion and the inner wall of the third annular connecting portion.

An endoscope includes the connecting structure for the bending section and the insertion tube described in any one of the foregoing.

According to the aforementioned endoscope, during the mounting process, the connecting tube is sleeved on the bending section, the first engaging portion is engaged with the second engaging portion, and the insertion tube is adhered to the connecting tube. Since the first engaging portion is provided in the circumferential direction of the bending section and the second engaging portion is provided in the circumferential direction of the connecting tube, the connecting tube is sleeved on the bending section while being connected to the bending section through engagement, which is beneficial to reduce the chances of the first engaging portion and the second engaging portion being broken by external force, and ensure the overall structural stability of the connecting structure for the bending section and the insertion tube. At the same time, by adhering the connecting tube to the insertion tube, the bending section is connected to the insertion tube in an indirect manner, which is conducive to avoiding the risk of the bending section falling off due to poor direct bonding stability, and further conducive to ensuring the connection stability between the bending section and the insertion tube, which reduces the probability of medical accidents caused by the bending section falling off from the insertion tube when the endoscope is in use, thereby improving the overall use quality of the endoscope.

### BRIEF DESCRIPTION OF THE DRAWINGS

The drawings constitute a part of the present disclosure and are used to provide a further understanding of the present utility model. The exemplary embodiments of the present utility model and their descriptions are used to explain the present utility model and do not constitute an improper limitation of the present utility model.

In order to illustrate the embodiments of the present utility model more clearly, the drawings used in the embodiments will be described briefly. Apparently, the following described drawings are merely for the embodiments of the present utility model, and other drawings can be derived by those of ordinary skill in the art without any creative effort.
FIG. 1 is a schematic view of a connecting structure for a bending section and an insertion tube after being mounted according to an embodiment.
FIG. 2 is an exploded view of the connecting structure for the bending section and the insertion tube according to an embodiment.

Description of reference signs:
100, connecting structure for a bending section and an insertion tube; 110, bending section; 111, first engaging portion; 112, engaging hole; 113, first engaging hole; 114, second engaging hole; 115, first annular connecting portion; 120, connecting tube; 121, second engaging portion; 122, engaging post; 123, first engaging post; 1231, guiding inclined surface; 124, second engaging post; 125, second annular connecting portion; 126, third annular connecting portion; 130, insertion tube; 131, fourth annular connecting portion.

### DETAILED DESCRIPTION OF THE EMBODIMENTS

In order to make the above objects, features and advantages of the present utility model clear and easier to understand, the specific embodiments of the present utility model are described in detail below in combination with the accompanying drawings. Many specific details are set forth in the following description to facilitate a full understanding of the present utility model. However, the present utility model can be implemented in many ways different from those described herein, and those skilled in the art can make similar improvements without departing from the connotation of the present utility model. Therefore, the present utility model is not limited by the specific embodiments disclosed below.

In the description of the present utility model, it should be understood that the terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential direction" are based on the azimuth or position relationship shown in the attached drawings, which are only for the convenience of describing the present utility model and simplifying the description, rather than indicating or implying that the device or element must have a specific azimuth, be constructed and operated in a specific azimuth, so such terms cannot be understood as a limitation of the present utility model.

In addition, the terms "first" and "second" are only used for descriptive purposes and cannot be understood as indicating or implying relative importance or implicitly indicating the number of indicated technical features. Thus, the features defined with "first" and "second" may explicitly or implicitly include at least one of the features. In the description of the present utility model, "a plurality of" means at least two, such as two, three, etc., unless otherwise expressly and specifically defined.

In the present utility model, unless otherwise expressly specified and limited, the terms "mount", "connect", "contact", "fix" and other terms should be understood in a broad sense, for example, they can be fixed connections, detachable connections, or integrated. They can be mechanical connection or electrical connection. They can be directly connected or indirectly connected through an intermediate medium. They can be the connection within two elements or the interaction relationship between two elements, unless otherwise expressly limited. For those skilled in the art, the specific meaning of the above terms in the present utility model can be understood according to the specific situation.

In the present utility model, unless otherwise expressly specified and limited, the first feature "above" or "below" the second feature may be in direct contact with the first and second features, or the first and second features may be in indirect contact through an intermediate medium. Moreover, the first feature is "above" the second feature, but the first feature is directly above or diagonally above the second feature, or it only means that the horizontal height of the first feature is higher than the second feature. The first feature is "below" of the second feature, which can mean that the first feature is directly below or obliquely below the second feature, or simply that the horizontal height of the first feature is less than that of the second feature.

It should be noted that when an element is called "fixed to" or "provided on" another element, it can be directly on another element or there can be a centered element. When an element is considered to be "connected" to another element, it can be directly connected to another element or there may be intermediate elements at the same time. The terms "vertical", "horizontal", "up", "down", "left", "right" and similar expressions used herein are for the purpose of illustration only and do not represent the only embodiment.

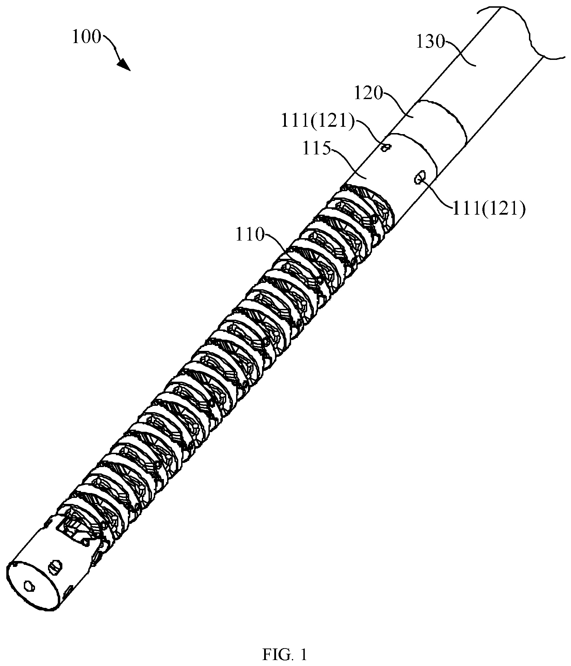

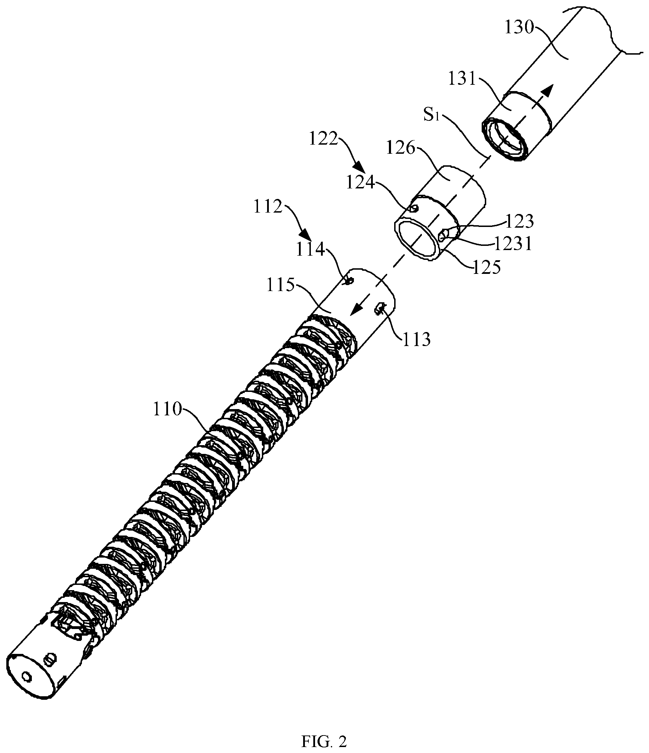

In an embodiment, referring to FIGs. 1 and 2, a connecting structure 100 for a bending section and an insertion tube includes a bending section 110, a connecting tube 120, and an insertion tube 130. The bending section 110 is provided with a first engaging portion 111 located in a circumferential direction of the bending section 110. The connecting tube 120 is sleeved with the bending section 110, and the connecting tube 120 is provided with a second engaging portion 121 located in a circumferential direction of the connecting tube 120. The first engaging portion 111 is engaged with the second engaging portion 121, so that the bending section 110 is connected to the connecting tube 120. The insertion tube 130 is adhered to the connecting tube 120.

According to the aforementioned connecting structure for the bending section and the insertion tube 100, during the mounting process, the connecting tube 120 is sleeved on the bending section 110, the first engaging portion 111 is engaged with the second engaging portion 121, and the insertion tube 130 is adhered to the connecting tube 120. Since the first engaging portion 111 is provided in the circumferential direction of the bending section 110 and the second engaging portion 121 is provided in the circumferential direction of the connecting tube 120, the connecting tube 120 is sleeved on the bending section 110 while being connected to the bending section 110 through engagement, which is beneficial to reduce the probability of the first engaging portion 111 and the second engaging portion 121 being broken by external force, and ensure the overall structural stability of the connecting structure for the bending section and the insertion tube 100. At the same time, by adhering the connecting tube 120 to the insertion tube 130, the bending section 110 is connected to the insertion tube 130 in an indirect manner, which is conducive to avoiding the risk of the bending section 110 falling off due to poor direct bonding stability, and further conducive to ensuring the connection stability between the bending section 110 and the insertion tube 130, which reduces the probability of medical accidents caused by the bending section 110 falling off from the insertion tube 130 when an endoscope is in use, thereby improving the overall use quality of the endoscope.

Further, referring to FIGs. 1 and 2, at least two first engaging portions 111 and at least two second engaging portions 121 are provided. The at least two first engaging portions 111 are spaced apart along the circumferential direction of the bending section 110. The at least two second engaging portions 121 are spaced apart along the circumferential direction of the connecting tube 120.

Specifically, referring to FIG. 2, four first engaging portions 111 are provided, and the four first engaging portions 111 are spaced apart along the circumferential direction of the bending section 110. Four second engaging portions 121 are provided, and the four second engaging portions 121 are spaced apart along the circumferential direction of the connecting tube 120. In this way, on the one hand, it is conducive to improving the operational convenience of the engagement connection and facilitating the mounting of the bending section 110 and the connecting tube 120. On the other hand, it is conducive to dispersing the stress at the connection point, improving the circumferential connection stability of the bending section 110 and the connecting tube 120, preventing the bending section 110 from loosening and falling, and thereby preventing the occurrence of medical accidents during the process of endoscopic examination. In this embodiment, only one selection of the numbers of the first bending section 111 and the second bending section 121 is provided, but not limited thereto.

Further, referring to FIG. 1 and FIG. 2, the at least two first engaging portions 111 are in one-to-one correspondence with the at least two second engaging portions 121. In this way, it is conducive to further improving the circumferential connection stability of the bending section 110 and the connecting tube 120.

In an embodiment (not shown), the first engaging portion 111 is an engaging post 122, and the second engaging portion 121 is an engaging hole 112. A cross-sectional profile of the engaging post 122 is matched with a shape of an opening of the engaging hole 112. In this way, the connection between the engaging post 122 and the engaging hole 112 is conducive to ensuring the stability of the engagement connection. At the same time, the engaging post 122 and the engaging hole 112 which are matched in shape with each other are conducive to avoiding loosening, thereby improving the overall quality of the endoscope.

Alternatively, in another embodiment, referring to FIGs. 1 and 2, the first engaging portion 111 is an engaging hole 112, the second engaging portion 121 is an engaging post 122, and a shape of an opening of the engaging hole 112 is matched with a cross-sectional profile of the engaging post 122. In this way, the connection between the engaging post 122 and the engaging hole 112 is conducive to ensuring the stability of the engagement connection. At the same time, the engaging post 122 and the engaging hole 112 which are adapted in shape are conducive to avoiding loosening, thereby improving the overall quality of the endoscope.

In an embodiment, referring to FIGs. 1 and 2, the engaging post 122 is provided with a guiding inclined surface. The guiding inclined surface is configured to guide the engaging post 122 to enter the engaging hole 112. In this way, when the bending section 110 is connected to the connecting tube 120, it is convenient for the engaging post 122 to enter the engaging hole 112, thereby improving the convenience of engagement connection between the connecting tube 120 and the bending section 110.

In an embodiment, referring to FIGs. 1 and 2, the at least two engaging posts 122 at least include a first engaging post 123 and a second engaging post 124, and the at least two engaging holes 112 at least include a first engaging hole 113 and a second engaging hole 114. The first engaging post 123 is engaged with the first engaging hole 113, and the second engaging post 124 is engaged with the second engaging hole 114. The cross-sectional profile of the first engaging post 123 is different from the cross-sectional profile of the second engaging post 124, and the shape of the opening of the first engaging hole 113 is different from the shape of the opening of the second engaging hole 114. In this way, the different shapes of the first engaging post 123 and the second engaging post 124 are used for distinction, which is conducive to directional connecting the bending section 110 to the connecting tube 120, avoiding the misalignment of the bending section 110 and the connecting tube 120, and is conducive to improving the overall quality of the endoscope.

Specifically, one first engaging post 123 and three second engaging posts 124 are provided. The one first engaging post 123 and the three second engaging posts 124 are spaced apart along the circumferential direction of the connecting tube 120. Correspondingly, one first engaging hole 113 and three second engaging holes 114 are provided. The one first engaging hole 113 and three second engaging holes 114 are spaced apart along the circumferential direction of the bending section 110. In this way, it is conducive to preventing the bending section 110 from being reversed during installation, thereby improving the convenience and efficiency of mounting the bending section 110.

In an embodiment, referring to FIGs. 1 and 2, the cross-sectional profile of the first engaging post 123 is oblong-shaped extending along an axial direction of the bending section 110, and the opening of the first engaging hole 113 is oblong-shaped extending along an axial direction of the connecting tube 120. The second engaging post 124 has a circular cross-sectional profile and the opening of the second engaging hole 114 is circular. In this embodiment, only one selection of the shapes of the engaging post 122 and the engaging hole 112 is provided, but not limited thereto.

In order to understand and illustrate the axial direction of the bending section 110, FIG. 2 is taken as an example. The axial direction of the bending section 110 is a direction pointed by any arrow on the straight line Si in FIG. 2. At the same time, the connecting tube 120 is coaxially connected to the bending section 110. The insertion tube 130 is coaxially connected to the connecting tube 120.

Specifically, referring to FIGs. 1 and 2, the bending section 110 is a POM (polymethanol) member, the connecting tube 120 is a PC (polycarbonate) tube, and the insertion tube 130 is a braided tube. In this way, the manufacturing material has low cost and is non-toxic and harmless, which, on the one hand, is conducive to reducing the production cost and improve the economic benefit of the product, on the other hand, it is conducive to avoiding secondary injuries such as allergies and improving the use quality of the endoscope. In this embodiment, only one material is selected for the bending section 110, the connecting tube 120 and the insertion tube 130, but not limited thereto.

In an embodiment, referring to FIG. 2, the bending section 110 is provided with a first annular connecting portion 115 connected to the bending section 110 and extending along the axial direction of the bending section 110. The first engaging portion 111 is located in a circumferential direction of the first annular connecting portion 115. The connecting tube 120 is provided with a second annular connecting portion 125 connected to the connecting tube 120 and extending along the axial direction of the connecting tube 120. The second engaging portion 121 is located in a circumferential direction of the second annular connecting portion 125. An outer diameter of the second annular connecting portion 125 is less than an inner diameter of the first annular connecting portion 115. The first annular connecting portion 115 is sleeved on the second annular connecting portion 125. The connecting tube 120 is engaged with the first annular connecting portion 115, and an outer wall of the connecting tube 120 is in smooth transition with an outer wall of the first annular connecting portion 115. In this way, after the bending section 110 and the connecting tube 120 are connected, the outer walls of the two have a smooth transition, which is conducive to improving the smoothness of the structure and avoiding damage to the organ tissue when entering human organs, thereby improving the overall use quality of the endoscope.

Further, a sealant is provided between the outer wall of the second annular connecting portion 125 and the inner wall of the first annular connecting portion 115. In this way, the first annular connecting portion 115 and the second annular connecting portion 125 are connected through engagement and adhesion, which is conducive to further improving the connection stability of the first bending section 110 and the connecting tube 120, thereby preventing the bending section 110 from falling off from the connecting tube 120 while ensuring sealing, thereby improving the overall use quality of the endoscope.

In another embodiment, the bending section 110 is provided with a first annular connecting portion 115 connected to the bending section 110 and extending along the axial direction of the bending section 110. The first engaging portion 111 is located in a circumferential direction of the first annular connecting portion 115. The connecting tube 120 is provided with a second annular connecting portion 125 connected to the connecting tube 120 and extending along the axial direction of the connecting tube 120. The second engaging portion 121 is located in a circumferential direction of the second annular connecting portion 125. An outer diameter of the first annular connecting portion 115 is less than an inner diameter of the second annular connecting portion 125. The second annular connecting portion 125 is sleeved on the first annular connecting portion 115. The bending section 110 is engaged with the second annular connecting portion 125, and an outer wall of the bending section 110 is in smooth transition with an outer wall of the second annular connecting portion 125. In this way, after the bending section 110 and the connecting tube 120 are connected, the outer walls of the two have a smooth transition, which is conducive to improving the smoothness of the structure and avoiding damage to the organ tissue when entering human organs, thereby improving the overall quality of the endoscope.

Further, a sealant is coated between the outer wall of the first annular connecting portion 115 and the inner wall of the second annular connecting portion 125. The first annular connecting portion 115 and the second annular connecting portion 125 are connected through a combination of engagement connection and adhesive bonding, which is conducive to further improving the connection stability of the first bending section 110 and the connecting tube 120, thereby preventing the bending section 110 from falling off from the connecting tube 120 while ensuring sealing, thereby improving the overall quality of the endoscope.

In an embodiment, referring to FIG. 2, the connecting tube 120 is further provided with a third annular connecting portion 126 connected to the connecting tube 120 and extending along the axial direction of the connecting tube 120. The second annular connecting portion 125 and the third annular connecting portion 126 are located at opposite ends of the connecting tube 120, respectively. The insertion tube 130 is provided with a fourth annular connecting portion 131 connected to the insertion tube 130 and extending along the axial direction of the insertion tube 130. An outer diameter of the fourth annular connecting portion 131 is less than an inner diameter of the third annular connecting portion 126. The third annular connecting portion 126 is sleeved on the fourth annular connecting portion 131. The insertion tube 130 is engaged with the third annular connecting portion 126, and the outer wall of the insertion tube 130 is in smooth transition with an outer wall of the third annular connecting portion 126. A structural adhesive is coated between the outer wall of the fourth annular connecting portion 131 and the inner wall of the third annular connecting portion 126. In this way, it is conducive to further ensuring the smoothness of the structure, avoiding damage to organ tissues when entering human organs, and thereby improving the overall use quality of the endoscope.

In an embodiment, an endoscope (not shown) includes the connecting structure 100 for the bending section and the insertion tube of the above embodiment.

According to the aforementioned endoscope, during the mounting process, the connecting tube 120 is sleeved on the bending section 110, the first engaging portion 111 is engaged with the second engaging portion 121, and the insertion tube 130 is adhered to the connecting tube 120. Since the first engaging portion 111 is provided in the circumferential direction of the bending section 110 and the second engaging portion 121 is provided in the circumferential direction of the connecting tube 120, the connecting tube 120 is sleeved on the bending section 110 while being connected to the bending section 110 through engagement, which is beneficial to reduce the probability of the first engaging portion 111 and the second engaging portion 121 being broken by external force, and ensure the overall structural stability of the connecting structure for the bending section and the insertion tube 100. At the same time, by adhering the connecting tube 120 to the insertion tube 130, the bending section 110 is connected to the insertion tube 130 in an indirect manner, which is conducive to avoiding the risk of the bending section 110 falling off due to poor direct bonding stability, and further conducive to ensuring the connection stability between the bending section 110 and the insertion tube 130, which reduces the probability of medical accidents caused by the bending section 110 falling off from the insertion tube 130 when the endoscope is in use, thereby improving the overall use quality of the endoscope.

## Claims

1. A connecting structure (100) for a bending section (110) and an insertion tube (130), comprising:
the bending section (110) provided with a first engaging portion (111), and the first engaging portion (111) being located in a circumferential direction of the bending section (110);
a connecting tube (120) sleeved with the bending section (110) and provided with a second engaging portion (121), the second engaging portion (121) being located in a circumferential direction of the connecting tube (120), the first engaging portion (111) being engaged with the second engaging portion (121) to enable the bending section (110) to be connected to the connecting tube (120); and
the insertion tube (130) adhered to the connecting tube (120);
wherein the bending section (110) is provided with a first annular connecting portion (115) connected to the bending section (110) and extending along an axial direction of the bending section (110), the first engaging portion (111) is located in a circumferential direction of the first annular connecting portion (115), the connecting tube (120) is provided with a second annular connecting portion (125) connected to the connecting tube (120) and extending along an axial direction of the connecting tube (120), the second engaging portion (121) is located in a circumferential direction of the second annular connecting portion (125);
an outer diameter of the second annular connecting portion (125) is less than an inner diameter of the first annular connecting portion (115), the first annular connecting portion (115) is sleeved on the second annular connecting portion (125), the connecting tube (120) is engaged with the first annular connecting portion (115), an outer wall of the connecting tube (120) is in smooth transition with an outer wall of the first annular connecting portion (115), and a sealant is coated between the outer wall of the second annular connecting portion (125) and the inner wall of the first annular connecting portion (115); or
an outer diameter of the first annular connecting portion (115) is less than an inner diameter of the second annular connecting portion (125), the second annular connecting portion (125) is sleeved on the first annular connecting portion (115), the bending section (110) is engaged with the second annular connecting portion (125), an outer wall of the bending section (110) is in smooth transition with an outer wall of the second annular connecting portion (125), and a sealant is coated between the outer wall of the first annular connecting portion (115) and the inner wall of the second annular connecting portion (125).

2. The connecting structure (100) for the bending section (110) and the insertion tube (130) according to claim 1, wherein at least two first engaging portions (111) and at least two second engaging portions (121) are provided, the at least two first engaging portions (111) are spaced apart along the circumferential direction of the bending section (110), the at least two second engaging portions (121) are spaced apart along a circumferential direction of the connecting tube (120), and the at least two first engaging portions (111) are in one-to-one correspondence with the at least two second engaging portions (121).

3. The connecting structure (100) for the bending section (110) and the insertion tube (130) according to claim 2, wherein the first engaging portion (111) is an engaging post (122), the second engaging portion (121) is an engaging hole (112), and a cross-sectional profile of the engaging post (122) is matched with a shape of an opening of the engaging hole (112); or
the first engaging portion (111) is an engaging hole (112), the second engaging portion (121) is an engaging post (122), and a shape of an opening of the engaging hole (112) is matched with a cross-sectional profile of the engaging post (122).

4. The connecting structure (100) for the bending section (110) and the insertion tube (130) according to claim 3, wherein the engaging post (122) is provided with a guiding inclined surface configured to guide the engaging post (122) to enter the engaging hole (112).

5. The connecting structure (100) for the bending section (110) and the insertion tube (130) according to claim 3, wherein the at least two of the engaging posts (122) at least comprise a first engaging post (123) and a second engaging post (124), the at least two engaging holes at least comprise a first engaging hole (113) and a second engaging hole (114), the first engaging post (123) is engaged with the first engaging hole (113), the second engaging post (124) is engaged with the second engaging hole (114), a cross-sectional profile of the first engaging post (123) is different from a cross-sectional profile of the second engaging post (124), and a shape of an opening of the first engaging hole (113) is different from a shape of an opening of the second engaging hole (114).

6. The connecting structure (100) for the bending section (110) and the insertion tube (130) according to claim 5, wherein the cross-sectional profile of the first engaging post (123) is oblong-shaped extending along an axial direction of the bending section (110), and the opening of the first engaging hole (113) is oblong-shaped extending along an axial direction of the connecting tube (120), the cross-sectional profile of the second engaging post (124) is circular, and the opening of the second engaging hole (114) is circular.

7. The connecting structure (100) for the bending section (110) and the insertion tube (30) according to claim 1, wherein the connecting tube (120) is further provided with a third annular connecting portion (126) connected to the connecting tube (120) and extending along the axial direction of the connecting tube (120), the second annular connecting portion (125) and the third annular connecting portion (126) are located at opposite ends of the connecting tube (120), respectively, the insertion tube (130) is provided with a fourth annular connecting portion (131) connected to the insertion tube (130) and extending along the axial direction of the insertion tube (130), an outer diameter of the fourth annular connecting portion (131) is less than an inner diameter of the third annular connecting portion (126), the third annular connecting portion (126) is sleeved on the fourth annular connecting portion (131), the insertion tube (130) is engaged with the third annular connecting portion (126), the outer wall of the insertion tube (130) is in smooth transition with an outer wall of the third annular connecting portion (126), and a structural adhesive is coated between the outer wall of the fourth annular connecting portion (131) and the inner wall of the third annular connecting portion (126).

8. An endoscope comprising the connecting structure (100) for the bending section (110) and the insertion tube (130) according to any one of claims 1 to 7.

## Patentansprüche

1. Eine Verbindungsstruktur (100) für einen Biegeabschnitt (110) und ein Einführrohr (130), die Folgendes beinhaltet:
den Biegeabschnitt (110), der mit einem ersten Eingriffsteil (111) bereitgestellt ist, und wobei sich der erste Eingriffsteil (111) in einer Umfangsrichtung des Biegeabschnitts (110) befindet;
ein Verbindungsrohr (120), das mit dem Biegeabschnitt (110) zusammengesteckt ist und mit einem zweiten Eingriffsteil (121) bereitgestellt ist, wobei sich der zweite Eingriffsteil (121) in einer Umfangsrichtung des Verbindungsrohrs (120) befindet, wobei der erste Eingriffsteil (111) mit dem zweiten Eingriffsteil (121) in Eingriff ist, um zu ermöglichen, dass der Biegeabschnitt (110) mit dem Verbindungsrohr (120) verbunden ist; und
das Einführrohr (130), das mit dem Verbindungsrohr (120) verklebt ist;
wobei der Biegeabschnitt (110) mit einem ersten ringförmigen Verbindungsteil (115) bereitgestellt ist, der mit dem Biegeabschnitt (110) verbunden ist und sich entlang einer axialen Richtung des Biegeabschnitts (110) erstreckt, sich der erste Eingriffsteil (111) in einer Umfangsrichtung des ersten ringförmigen Verbindungsteils (115) befindet, das Verbindungsrohr (120) mit einem zweiten ringförmigen Verbindungsteil (125) bereitgestellt ist, der mit dem Verbindungsrohr (120) verbunden ist und sich entlang einer axialen Richtung des Verbindungsrohrs (120) erstreckt, sich der zweite Eingriffsteil (121) in einer Umfangsrichtung des zweiten ringförmigen Verbindungsteils (125) befindet;
ein Außendurchmesser des zweiten ringförmigen Verbindungsteils (125) kleiner als ein Innendurchmesser des ersten ringförmigen Verbindungsteils (115) ist, der erste ringförmige Verbindungsteil (115) auf den zweiten ringförmigen Verbindungsteil (125) aufgesteckt ist, das Verbindungsrohr (120) mit dem ersten ringförmigen Verbindungsteil (115) in Eingriff ist, eine Außenwand des Verbindungsrohrs (120) einen nahtlosen Übergang zu einer Außenwand des ersten ringförmigen Verbindungsteils (115) bildet und zwischen der Außenwand des zweiten ringförmigen Verbindungsteils (125) und der Innenwand des ersten ringförmigen Verbindungsteils (115) ein Dichtungsmittel aufgetragen ist; oder
ein Außendurchmesser des ersten ringförmigen Verbindungsteils (115) kleiner als ein Innendurchmesser des zweiten ringförmigen Verbindungsteils (125) ist, der zweite ringförmige Verbindungsteil (125) auf den ersten ringförmigen Verbindungsteil (115) aufgesteckt ist, der Biegeabschnitt (110) mit dem zweiten ringförmigen Verbindungsteil (125) in Eingriff ist, eine Außenwand des Biegeabschnitts (110) einen nahtlosen Übergang zu einer Außenwand des zweiten ringförmigen Verbindungsteils (125) bildet und zwischen der Außenwand des ersten ringförmigen Verbindungsteils (115) und der Innenwand des zweiten ringförmigen Verbindungsteils (125) ein Dichtungsmittel aufgetragen ist.

2. Verbindungsstruktur (100) für den Biegeabschnitt (110) und das Einführrohr (130) gemäß Anspruch 1, wobei mindestens zwei erste Eingriffsteile (111) und mindestens zwei zweite Eingriffsteile (121) bereitgestellt sind, die mindestens zwei ersten Eingriffsteile (111) entlang der Umfangsrichtung des Biegeabschnitts (110) voneinander beabstandet sind, die mindestens zwei zweiten Eingriffsteile (121) entlang einer Umfangsrichtung des Verbindungsrohrs (120) voneinander beabstandet sind und die mindestens zwei ersten Eingriffsteile (111) zu den mindestens zwei zweiten Eingriffsteilen (121) in einer Eins-zu-Eins-Entsprechung stehen.

3. Verbindungsstruktur (100) für den Biegeabschnitt (110) und das Einführrohr (130) gemäß Anspruch 2, wobei der erste Eingriffsteil (111) ein Eingriffsstift (122) ist, der zweite Eingriffsteil (121) ein Eingriffsloch (112) ist und ein Querschnittsprofil des Eingriffsstifts (122) mit einer Form einer Öffnung des Eingriffslochs (112) übereinstimmt; oder
der erste Eingriffsteil (111) ein Eingriffsloch (112) ist, der zweite Eingriffsteil (121) ein Eingriffsstift (122) ist und eine Form einer Öffnung des Eingriffslochs (112) mit einem Querschnittsprofil des Eingriffsstifts (122) übereinstimmt.

4. Verbindungsstruktur (100) für den Biegeabschnitt (110) und das Einführrohr (130) gemäß Anspruch 3, wobei der Eingriffsstift (122) mit einer geneigten Führungsfläche bereitgestellt ist, die konfiguriert ist, um den Eingriffsstift (122) zu führen, damit dieser in das Eingriffsloch (112) eintritt.

5. Verbindungsstruktur (100) für den Biegeabschnitt (110) und das Einführrohr (130) gemäß Anspruch 3, wobei die mindestens zwei der Eingriffsstifte (122) mindestens einen ersten Eingriffsstift (123) und einen zweiten Eingriffsstift (124) beinhalten, die mindestens zwei Eingriffslöcher mindestens ein erstes Eingriffsloch (113) und ein zweites Eingriffsloch (114) beinhalten, der erste Eingriffsstift (123) mit dem ersten Eingriffsloch (113) in Eingriff ist, der zweite Eingriffsstift (124) mit dem zweiten Eingriffsloch (114) in Eingriff ist, sich ein Querschnittsprofil des ersten Eingriffsstifts (123) von einem Querschnittsprofil des zweiten Eingriffsstifts (124) unterscheidet und sich eine Form einer Öffnung des ersten Eingriffslochs (113) von einer Form einer Öffnung des zweiten Eingriffslochs (114) unterscheidet.

6. Verbindungsstruktur (100) für den Biegeabschnitt (110) und das Einführrohr (130) gemäß Anspruch 5, wobei das Querschnittsprofil des ersten Eingriffsstifts (123) länglich geformt ist und sich entlang einer axialen Richtung des Biegeabschnitts (110) erstreckt und die Öffnung des ersten Eingriffslochs (113) länglich geformt ist und sich entlang einer axialen Richtung des Verbindungsrohrs (120) erstreckt, das Querschnittsprofil des zweiten Eingriffsstifts (124) kreisförmig ist und die Öffnung des zweiten Eingriffslochs (114) kreisförmig ist.

7. Verbindungsstruktur (100) für den Biegeabschnitt (110) und das Einführrohr (30) gemäß Anspruch 1, wobei das Verbindungsrohr (120) ferner mit einem dritten ringförmigen Verbindungsteil (126) bereitgestellt ist, der mit dem Verbindungsrohr (120) verbunden ist und sich entlang der axialen Richtung des Verbindungsrohrs (120) erstreckt, sich der zweite ringförmige Verbindungsteil (125) und der dritte ringförmige Verbindungsteil (126) jeweils an entgegengesetzten Enden des Verbindungsrohrs (120) befinden, das Einführrohr (130) mit einem vierten ringförmigen Verbindungsteil (131) bereitgestellt ist, der mit dem Einführrohr (130) verbunden ist und sich entlang der axialen Richtung des Einführrohrs (130) erstreckt, ein Außendurchmesser des vierten ringförmigen Verbindungsteils (131) kleiner als ein Innendurchmesser des dritten ringförmigen Verbindungsteils (126) ist, der dritte ringförmige Verbindungsteil (126) auf den vierten ringförmigen Verbindungsteil (131) aufgesteckt ist, das Einführrohr (130) mit dem dritten ringförmigen Verbindungsteil (126) in Eingriff ist, die Außenwand des Einführrohrs (130) mit einer Außenwand des dritten ringförmigen Verbindungsteils (126) einen nahtlosen Übergang bildet und zwischen der Außenwand des vierten ringförmigen Verbindungsteils (131) und der Innenwand des dritten ringförmigen Verbindungsteils (126) ein Strukturklebstoff aufgetragen ist.

8. Ein Endoskop, das die Verbindungsstruktur (100) für den Biegeabschnitt (110) und das Einführrohr (130) gemäß einem der Ansprüche 1 bis 7 beinhaltet.

## Revendications

1. Une structure de raccordement (100) pour une section de courbure (110) et un tube d'insertion (130), comprenant :
la section de courbure (110) pourvue d'une première portion de mise en prise (111), la première portion de mise en prise (111) étant située dans une direction circonférentielle de la section de courbure (110) ;
un tube de raccordement (120) emmanché avec la section de courbure (110) et pourvu d'une deuxième portion de mise en prise (121), la deuxième portion de mise en prise (121) étant située dans une direction circonférentielle du tube de raccordement (120), la première portion de mise en prise (111) étant en prise avec la deuxième portion de mise en prise (121) afin de permettre à la section de courbure (110) d'être raccordée au tube de raccordement (120) ; et
le tube d'insertion (130) adhérent au tube de raccordement (120) ;
dans laquelle la section de courbure (110) est pourvue d'une première portion de raccordement annulaire (115) raccordée à la section de courbure (110) et s'étendant le long d'une direction axiale de la section de courbure (110), la première portion de mise en prise (111) est située dans une direction circonférentielle de la première portion de raccordement annulaire (115), le tube de raccordement (120) est pourvu d'une deuxième portion de raccordement annulaire (125) raccordée au tube de raccordement (120) et s'étendant le long d'une direction axiale du tube de raccordement (120), la deuxième portion de mise en prise (121) est située dans une direction circonférentielle de la deuxième portion de raccordement annulaire (125) ;
un diamètre externe de la deuxième portion de raccordement annulaire (125) est inférieur à un diamètre interne de la première portion de raccordement annulaire (115), la première portion de raccordement annulaire (115) est emmanchée sur la deuxième portion de raccordement annulaire (125), le tube de raccordement (120) est en prise avec la première portion de raccordement annulaire (115), une paroi externe du tube de raccordement (120) est en transition lisse avec une paroi externe de la première portion de raccordement annulaire (115), et un matériau d'étanchéité est revêtu entre la paroi externe de la deuxième portion de raccordement annulaire (125) et la paroi interne de la première portion de raccordement annulaire (115) ; ou
un diamètre externe de la première portion de raccordement annulaire (115) est inférieur à un diamètre interne de la deuxième portion de raccordement annulaire (125), la deuxième portion de raccordement annulaire (125) est emmanchée sur la première portion de raccordement annulaire (115), la section de courbure (110) est en prise avec la deuxième portion de raccordement annulaire (125), une paroi externe de la section de courbure (110) est en transition lisse avec une paroi externe de la deuxième portion de raccordement annulaire (125), et un matériau d'étanchéité est revêtu entre la paroi externe de la première portion de raccordement annulaire (115) et la paroi interne de la deuxième portion de raccordement annulaire (125).

2. La structure de raccordement (100) pour la section de courbure (110) et le tube d'insertion (130) selon la revendication 1, dans laquelle au moins deux premières portions de mise en prise (111) et au moins deux deuxièmes portions de mise en prise (121) sont fournies, les au moins deux premières portions de mise en prise (111) sont espacées le long de la direction circonférentielle de la section de courbure (110), les au moins deux deuxièmes portions de mise en prise (121) sont espacées le long d'une direction circonférentielle du tube de raccordement (120), et les au moins deux premières portions de mise en prise (111) sont dans une correspondance biunivoque avec les au moins deux deuxièmes portions de mise en prise (121).

3. La structure de raccordement (100) pour la section de courbure (110) et le tube d'insertion (130) selon la revendication 2, dans laquelle la première portion de mise en prise (111) est un montant de mise en prise (122), la deuxième portion de mise en prise (121) est un trou de mise en prise (112), et un profil de section transversale du montant de mise en prise (122) est mis en correspondance avec une forme d'une ouverture du trou de mise en prise (112) ; ou
la première portion de mise en prise (111) est un trou de mise en prise (112), la deuxième portion de mise en prise (121) est un montant de mise en prise (122), et une forme d'une ouverture du trou de mise en prise (112) est mise en correspondance avec un profil de section transversale du montant de mise en prise (122).

4. La structure de raccordement (100) pour la section de courbure (110) et le tube d'insertion (130) selon la revendication 3, dans laquelle le montant de mise en prise (122) est pourvu d'une surface inclinée de guidage configurée pour guider le montant de mise en prise (122) afin qu'il entre dans le trou de mise en prise (112).

5. La structure de raccordement (100) pour la section de courbure (110) et le tube d'insertion (130) selon la revendication 3, dans laquelle les au moins deux montants de mise en prise (122) comprennent au moins un premier montant de mise en prise (123) et un deuxième montant de mise en prise (124), les au moins deux trous de mise en prise comprennent au moins un premier trou de mise en prise (113) et un deuxième trou de mise en prise (114), le premier montant de mise en prise (123) est en prise avec le premier trou de mise en prise (113), le deuxième montant de mise en prise (124) est en prise avec le deuxième trou de mise en prise (114), un profil de section transversale du premier montant de mise en prise (123) est différent d'un profil de section transversale du deuxième montant de mise en prise (124), et une forme d'une ouverture du premier trou de mise en prise (113) est différente d'une forme d'une ouverture du deuxième trou de mise en prise (114).

6. La structure de raccordement (100) pour la section de courbure (110) et le tube d'insertion (130) selon la revendication 5, dans laquelle le profil de section transversale du premier montant de mise en prise (123) est de forme oblongue s'étendant le long d'une direction axiale de la section de courbure (110), et l'ouverture du premier trou de mise en prise (113) est de forme oblongue s'étendant le long d'une direction axiale du tube de raccordement (120), le profil de section transversale du deuxième montant de mise en prise (124) est circulaire, et l'ouverture du deuxième trou de mise en prise (114) est circulaire.

7. La structure de raccordement (100) pour la section de courbure (110) et le tube d'insertion (30) selon la revendication 1, dans laquelle le tube de raccordement (120) est en outre pourvu d'une troisième portion de raccordement annulaire (126) raccordée au tube de raccordement (120) et s'étendant le long de la direction axiale du tube de raccordement (120), la deuxième portion de raccordement annulaire (125) et la troisième portion de raccordement annulaire (126) sont situées au niveau d'extrémités opposées du tube de raccordement (120), respectivement, le tube d'insertion (130) est pourvu d'une quatrième portion de raccordement annulaire (131) raccordée au tube d'insertion (130) et s'étendant le long d'une direction axiale du tube d'insertion (130), un diamètre externe de la quatrième portion de raccordement annulaire (131) est inférieur à un diamètre interne de la troisième portion de raccordement annulaire (126), la troisième portion de raccordement annulaire (126) est emmanchée sur la quatrième portion de raccordement annulaire (131), le tube d'insertion (130) est en prise avec la troisième portion de raccordement annulaire (126), la paroi externe du tube d'insertion (130) est en transition lisse avec une paroi externe de la troisième portion de raccordement annulaire (126), et un adhésif structural est revêtu entre la paroi externe de la quatrième portion de raccordement annulaire (131) et la paroi interne de la troisième portion de raccordement annulaire (126).

8. Un endoscope comprenant la structure de raccordement (100) pour la section de courbure (110) et le tube d'insertion (130) selon l'une quelconque des revendications 1 à 7.
